# EUROPEAN PATENT SPECIFICATION

(11) **EP 1 113 009 B1**
(45) Date of publication and mention of the grant of the patent: **30.03.2005**
(21) Application number: 00127206.1
(22) Date of filing: 14.12.2000
(51) Int. Cl.: C07D 249/12

(54) **A process for the manufacture of the salts of sulfonylaminocarbonyl triazolinones**
Verfahren zur Herstellung von den Sulfonylaminocarbonyl-Triazolinonsalzen
Procédé de préparation des sels de sulphonylaminocarbonyl-triazolinones

(30) Priority: 27.12.1999 US 472335; 27.12.1999 US 472672
(43) Date of publication of application: 04.07.2001
(73) Proprietor: Bayer Corporation, Pittsburgh, PA 15205 (US)
(72) Inventor: Prasad, Vidyanatha A., Leawood, KS 66224 (US); Kulkarni, Shekhar V., Shawnee, KS 66215 (US); Rivadeneira, Eric, Dr., Overland Park, KS 66213 (US); Desai, Vijay C., Shawnee, KS 66216 (US); Jelich, Klaus, Dr., 42113 Wuppertal (DE)
(74) Representative: Bader, Axel Jochen

(56) References cited:
- US-A- 5 405 970
- US-A- 5 534 486
- US-A- 5 750 718
- US-A- 5 869 681
- US-A- 6 147 221
- US-A- 6 147 222
- US-A- 6 160 125

## Description

The present invention relates to improvements in the process for manufacturing the salts of certain sulfonylaminocarbonyl triazolinones, which are herbicidally active compounds (e.g. known from US 5,534,468).

A new process, wherein a sulfonylaminocarbonyl triazolinone (which is obtained in a previous process step with or without isolation) is converted under pH-controlled conditions to a salt thereof is described.

The invention is explained in detail as follows:

Sulfonylaminocarbonyl triazolinones are well known in the art, as are processes for their preparation and their use as herbicides. European Patent EP-A 341,489 discloses certain substituted sulfonylamino-carbonyl triazolinones having herbicidal properties. Further, U.S. Patents 5,534,486 and 5,869,681 describe a process for producing sulfonylaminocarbonyl triazolinones which are bonded via oxygen. The process includes the reaction of a triazolinone with a sulfonamide derivative. U.S. Patent 5,750,718 describes intermediates for herbicidal sulfonylaminocarbonyl triazolinones having substituents which are bonded via sulfur.

However, the known prior art processes produce sulfonylaminocarbonyl triazolinones in unsatisfactory yield and purity. Thus, there is a need in the art for a process to manufacture sulfonylaminocarbonyl triazolinones in high yield and purity.

The present invention is related to a process for the preparation of the salts of sulfonylaminocarbonyl triazolinones. The process comprises the step of:
reacting an intermediate product of general formula (III) 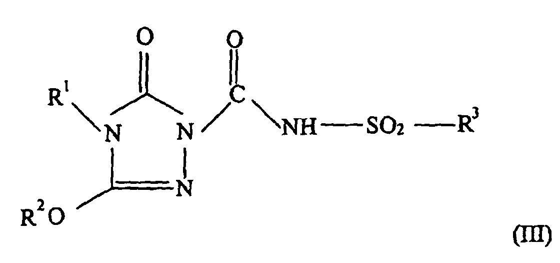 wherein
- R¹: represents alkyl, alkenyl or alkynyl having in each case up to 6 carbon atoms, and each of which is unsubstituted or substituted by cyano, halogen, or C₁-C₄-alkoxy,
or
represents cycloalkyl group having 3 to 6 carbon atoms or cycloalkylalkyl group having 3 to 6 carbon atoms in the cycloalkyl moiety and 1 to 4 carbon atoms in the alkyl moiety, each of which is unsubstituted or substituted by halogen or C₁-C₄-alkyl,
or
represents aryl group having 6 or 10 carbon atoms or arylalkyl group having 6 or 10 carbon atoms in the aryl moiety and 1 to 4 carbon atoms in the alkyl moiety, each of which is unsubstituted or substituted by carboxyl, nitro, cyano, halogen, C₁-C₄-alkyl, C₁-C₄-halogenoalkyl, C₁-C₄-alkoxy, C₁-C₄-halogenoalkoxy or C₁-C₄-alkoxy-carbonyl. and
- R²: represents alkyl, alkenyl or alkenyl, each of which has up to 6 carbon atoms, and each of which is unsubstituted or substituted by cyano, halogen or C₁-C₄-alkoxy,
or
represents cycloalkyl having 3 to 6 carbon atoms or cycloalkylalkyl having 3 to 6 carbon atoms in the cycloalkyl moiety and 1 to 4 carbon atoms in the alkyl moiety, each of which is unsubstituted or substituted by halogen or C₁-C₄ -alkyl,
or
represents aryl having 6 to 10 carbon atoms or arylalkyl having 6 or 10 carbon atoms in the aryl moiety and 1 to 4 carbon atoms in the alkyle moiety, each of which is unsubstituted or substituted by carboxyl, cyano, nitro, halogen, C₁-C₄-alkyl, C₁-C₄-halogenoalkyl, C₁-C₄-alkoxy, C₁-C₄-halogenoalkoxy or C₁-C₄-alkoxy-carbonyl, and
- R³: represents the group 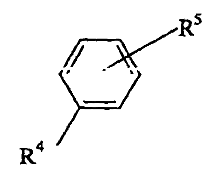
wherein
- R⁴ and R⁵: are identical or different and represent hydrogen, fluorine, chlorine, bromine, iodine, nitro, C₁-C₆-alkyl, which is unsubstituted or substituted by fluorine, chlorine, bromine, cyano, carboxyl, C₁-C₄-alkoxycarbonyl, C₁-C₄-alkylaminocarbonyl, di-(C₁-C₄-alkyl)aminocarbonyl, hydroxyl, C₁-C₄-alkoxy, formyloxy, C₁-C₄-alkylcarbonyloxy, C₁-C₄-alkoxycarbonyloxy, C₁-C₄-alkylaminocarbonyloxy, C₁-C₄-alkylthio, C₁-C₄-alkylsulfinyl, C₁-C₄-alkylsulfonyl, di-(C₁-C₄-alkyl)-aminosulfonyl, C₃-C₆-cycloalkyl or phenyl,
or
represent C₂-C₆-alkenyl which is unsubstituted or substituted by fluorine, chlorine, bromine, cyano, C₁-C₄-alkoxycarbonyl, carboxyl or phenyl,
or
represent C₂-C₆-alkynyl which is unsubstituted or substituted by fluorine, chlorine, bromine, cyano, C₁ -C₄-alkoxycarbonyl, carboxyl or phenyl,
or
represent C₁-C₄-alkoxy which is unsubstituted or substituted by fluorine, chlorine, bromine, cyano, carboxyl, C₁-C₄-alkoxycarbonyl, C₁-C₄-alkoxy, C₁-C₄-alkylthio, C₁-C₄ -alkylsulfinyl or C₁-C₄-alkylsulfonyl,
or
represent C₁-C₄-alkylthio which is unsubstituted or substituted by fluorine, chlorine, bromine, cyano, carboxyl, C₁-C₄-alkoxycarbonyl, C₁-C₄-alkylthio, C₁-C₄ -alkylsulfinyl or C₁-C₄-alkylsulfonyl,
or
represent C₃-C₆ alkenyloxy which is unsubstituted or substituted by fluorine, chlorine, bromine, cyano or C₁-C₄-alkoxycarbonyl,
or
represent C₂-C₆-alkenylthio which is unsubstituted or substituted by fluorine, chlorine, bromine, cyano, nitro,
C₁-C₃-aklylthio or C₁-C₄-akoxycarbonyl, C₃-C₆-alkynyloxy, C₃-C₆-alkynylthio or the radical -S(O)ₚ-R⁶ where p represents the numbers 1 or 2, and
- R⁶: represents C₁C₄-alkyl which is unsubstituted or substituted by fluorine, chlorine, bromine, cyano or C₁-C₄-alkoxy-carbonyl, C₃-C₆-alkenyl, C₃-C₆-alkynyl, C₁-C₄-alkoxy, C₁-C₄-alkoxy-C₁-C₄-alkylamino, C₁-C₄-alkylamino, di-(C₁-C₄-alkyl)amino, phenyl or the radical ―NHOR⁷
wherein
- R⁷: represents C₁-C₁₂-alkyl which is unsubstituted or substituted by fluorine, chlorine, cyano, C₁-C₄-alkoxy, C₁-C₄-alkylthio, C₁-C₄-alkylsulfinyl, C₁-C₄-alkylsulfonyl, C₁-C₄-alkyl-carbonyl, C₁-C₄-alkoxy-carbonyl, C₁-C₄-alkylamino-carbonyl or di-(C₁-C₄-alkyl)-amino-carbonyl), or represents C₃-C₆-alkenyl which is unsubstituted or substituted by fluorine, chlorine or bromine, C₃-C₆-alkynyl, C₃-C₆-cycloalkyl, C₃-C₆-cycloalkyl-C₁-C₂-alkyl, phenyl-C₁-C₂-alkyl which is unsubstituted or substituted by fluorine, chlorine, nitro, cyano, C₁-C₄-alkyl, C₁-C₄-alkoxy or C₁-C₄-alkoxy-carbonyl), or represents benzhydryl, or represents phenyl which is unsubstituted or substituted by fluorine, chlorine, nitro, cyano, C₁-C₄-alkyl) trifluoromethyl, C₁-C₄-alkoxy, C₁-C₂-fluoroalkoxy, C₁-C₄-alkylthio, trifluoromethylthio or C₁-C₄-alkoxy-carbonyl,
- R⁴ and/or R⁵: furthermore represent phenyl or phenoxy, or represent C₁-C₄-alkylcarbonylamino, C₁-C₄ -alkoxycarbonyl-amino, C₁-C₄-alkylamino-carbonyl-amino, di-(C₁-C₄-alkyl)-amino-carbonyl-amino, or the radical -CO-R⁸
wherein
- R⁸: represents C₁-C₆-alkyl, C₁-C₆-alkoxy, C₃-C₆-cycloakoxy, C₃-C₆-alkenyloxy, C₁-C₄-alkylthio, C₁-C₄-alkylamino, C₁-C₄-alkoxyamino. C₁-C₄-alkoxy-C₁-C₄-alkyl-amino or di-(C₁-C₄-alkyl)-amino which are unsubstituted or substituted by halogen selected from the group consisting of fluorine and chlorine,
- R⁴ and/or R⁵: furthermore represent trimethylsilyl, thiazolinyl, C₁-C₄-alkylsulfonyloxy, di-(C₁-C₄-alkyl)-aminosulfonylamino or the radical -CH=N-R⁹
wherein
- R⁹: represents C₁-C₆-alkyl which is unsubstituted or substituted by fluorine, chlorine, cyano, carboxyl, C₁-C₄-alkoxy, C₁-C₄-alkylthio, C₁-C₄-alkylsulfinyl or C₁-C₄-alkylsulfonyl, or represents benzyl which is unsubstituted or substituted by fluorine or chlorine, or represents C₃-C₆-alkenyl or C₃-C₆-alkynyl, each of which is unsubstituted or substituted by fluorine or chlorine, or represents phenyl which is unsubstituted or substituted by fluorine, chlorine, bromine, C₁-C₄-alkyl, C₁-C₄-alkoxy, trifluoromethyl, trifluoromethoxy or trifluoromethylthio, or represents unsubstituted or halogen substituted C₁-C₆-alkoxy, C₃-C₆-alkenoxy, C₃-C₆-alkynoxy or benzyloxy, wherein the halogen is selected from the group consisting of fluorine and chlorine, or represents amino, C₁-C₄-alkylamino, di-(C₁-C₄-alkyl)-amino, phenylamino, C₁-C₄-alkyl-carbonylamino, C₁-C₄-alkoxy-carbonylamino or C₁- C₄-alkyl-sulfonylamino, or represents phenylsulfonylamino which is unsubstituted or substituted by fluorine, chlorine, bromine or methyl,
furthermore
- R³: represents the radical 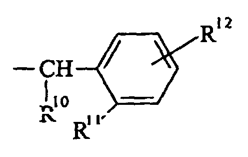
wherein
- R¹⁰: represents hydrogen or C₁-C₄-alkyl,
wherein
- R¹¹ and R¹²: are identical or different and represent hydrogen, fluorine, chlorine, bromine, nitro, cyano, C₁-C₄-alkyl which is unsubstituted or substituted by halogen selected from the group consisting of fluorine and chlorine, C₁-C₄-alkoxy which is unsubstituted or substituted by halogen selected from the group consisting of fluorine and chlorine, carboxyl, C₁-C₄-alkoxy-carbonyl, dimethylaminocarbonyl, C₁-C₄-alkylsulfonyl or di-(C₁-C₄-alkyl)-aminosulfonyl;
furthermore
- R³: represents the radical 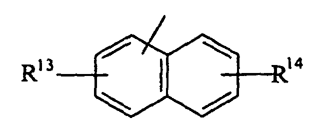
wherein
- R¹³ and R¹⁴: are identical or different and represent hydrogen, fluorine, chlorine, bromine, nitro, cyano, C₁-C₄-alkyl which is unsubstituted or substituted by halogen selected from the group consisting of fluorine and chlorine, or C₁-C₄-alkoxy which is unsubstituted or substituted by halogen selected from the group consisting of fluorine and chlorine;
furthermore
- R³: represents the radical 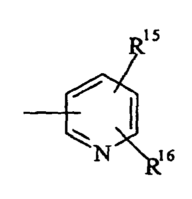
wherein
- R¹⁵ and R¹⁶: are identical or different and represent hydrogen, fluorine, chlorine, bromine, nitro, cyano, C₁-C₄-alkyl which is unsubstituted or substituted by halogen selected from the group consisting of fluorine and chlorine, C₁-C₄-alkoxy which is unsubstituted or substituted by halogen selected from the group consisting of fluorine and chlorine, or represent C₁-C₄-alkylthio, C₁-C₄-alkylsulfinyl or C₁C₄-alkylsulfonyl which are unsubstituted or substituted by halogen selected from the group consisting of fluorine and chlorine, or represent aminosulfonyl, mono-(C₁-C₄-alkyl)-aminosulfonyl, di-(C₁-C₄-alkyl)-aminosulfonyl or C₁-C₄-akoxycarbonyl or dimethylaminocarbonyl;
furthermore
- R³: represents the radical 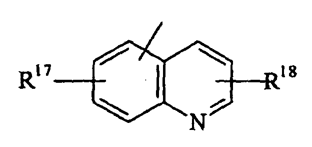
wherein
- R¹⁷ and R¹⁸: are identical or different and represent hydrogen, fluorine, chlorine, bromine, C₁-C₄-alkyl which is unsubstituted or substituted by halogen selected from the group consistmg of fluorine and bromine, C₁-C₄-alkoxy which is unsubstituted or substituted by halogen selected from the group consisting of fluorine and chlorine, C₁-C₄-alkylthio, C₁-C₄-akylsulfinyl or C₁-C₄-alkylsulfonyl which are unsubstituted or substituted by halogen selected from the group consisting of fluorine and chlorine, or represent di-(C₁-C₄-alkyl)-aminosulphonyl;
furthermore
- R³: represents the radical 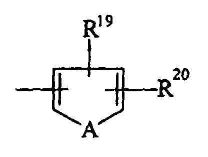
wherein
- R¹⁹ and R²⁰: are identical or different and represent hydrogen, fluorine, chlorine, bromine, cyano, nitro, C₁-C₄-alkyl which is unsubstituted or substituted by halogen selected from the group consisting of fluorine and chlorine, C₁-C₄-alkoxy which is unsubstituted or substituted by halogen selected from the group consisting of fluorine and chlorine, C₁-C₄-alkylthio, C₁-C₄-alkylsulfinyl or C₁-C₄-alkysulfonyl which is unsubstituted or substituted by halogen selected from the group consisting of fluorine and chlorine, di-(C₁-C₄-akyl)-aminosulfonyl, C₁-C₄-alkoxy-carbonyl or dimethylaminocarbonyl, and
- A: represents oxygen, sulfur or the group N―Z¹,
wherein
- Z¹: represents hydrogen, C₁-C₄-alkyl which is unsubstituted or substituted by fluorine, chlorine, bromine, or cyano, C₃-C₆-cycloalkyl, benzyl, phenyl which is unsubstituted or substituted by fluorine, chlorine, bromine or nitro, C₁-C₄-alkylcarbonyl, C₁-C₄-alkoxy-carbonyl or di-(C₁-C₄-alkyl)-amino-carbonyl;
furthermore
- R³: represents the radical 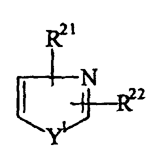
wherein
- R²¹ and R²²: are identical or different and represent hydrogen, C₁-C₄-alkyl, halogen, C₁-C₄-alkoxycarbonyl, C₁-C₄-alkoxy or C₁-C₄-halogenoalkoxy,
- Y¹: represents sulfur or the group N-R²³,
wherein
- R²³: represents hydrogen or C₁-C₄-alkyl;
furthermore
- R³: represents the radical 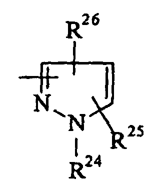
wherein
- R²⁴: represents hydrogen, C₁-C₄-alkyl, benzyl, pyridyl, quinolinyl or phenyl,
- R²⁵: represents hydrogen, halogen, cyano, nitro, C₁-C₄-alkyl which is unsubstituted or substituted by halogen selected from the group consisting of fluorine and chlorine, C₁-C₄-alkoxy which is unsubstituted or substituted by halogen selected from the group consisting of fluorine and chlorine, dioxolanyl or C₁-C₄-alkoxy-carbonyl, and
- R²⁶: represents hydrogen, halogen or C₁-C₄-alkyl;
furthermore
- R³: represents a compound selected from the group consisting of 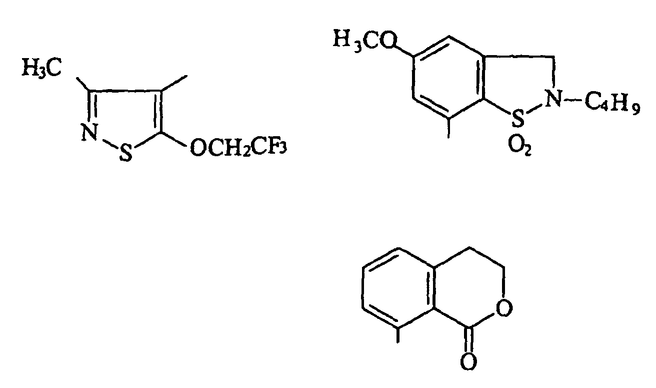
under pH controlled conditions with a base in such way that the base is added in an amount such that the pH of the mixture attained is from 5 to 9, to produce a salt thereof, a final product of the general formula (IV) 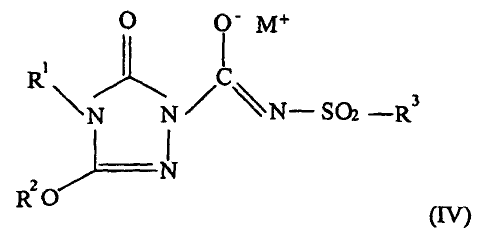 wherein
R¹, R², and R³ are as defined above, and M represents an alkali
or alkaline earth metal or protonated ammonia derivative.

More preferably,
- R¹: represents methyl, ethyl, n- or i-propyl, n-, i-, s- or t-butyl, each of which is unsubstituted or substituted by cyano, fluorine, chlorine or bromine, methoxy or ethoxy,
or
represents propenyl, butenyl, propinyl or butinyl, each of which is unsubstituted or substituted by cyano, fluorine, chlorine or bromine,
or
represents cyclopropyl, cyclobutyl or cyclopropylmethyl, each of which is unsubstituted or substituted by fluorine, chlorine, bromine, methyl or ethyl,
or
represents phenyl or benzyl, each of which is unsubstituted or substituted by cyano, fluorine, chlorine, bromine, methyl, ethyl, trifluoromethyl, methoxy, ethoxy,
difluoromethoxy, trifluoromethoxy, methoxycarbonyl or
ethoxycarbonyl.

Most preferably,
- R¹: represents methyl.

More preferably,
- R²: represents methyl, ethyl, n- or i-propyl or n-, i-, s- or t-butyl, each of which is unsubstituted or substituted by cyano, fluorine, chlorine or bromine, methoxy or ethoxy,
or
represents propenyl, butenyl, propinyl or butinyl, each of which is unsubstituted or substituted by cyano, fluorine, chlorine or bromine,
or
represents cyclopropyl, or cyclopropylmethyl, each of which is unsubstituted or substituted by fluorine, chlorine, bromine, methyl or ethyl,
or
represents phenyl or benzyl, each of which is unsubstituted or substituted by cyano, fluorine, chlorine, bromine, methyl, ethyl, trifluoromethyl, methoxy, ethoxy, difluoromethoxy, trifluoromethoxy, methoxycarbonyl or ethoxycarbonyl.

Most preferably,
- R²: represents methyl, n- or i-propyl.

In particular, the invention relates to compounds of the formula (IV) wherein
- R¹: represents C₁-C₄-alkyl which is unsubstituted or substituted by fluorine, cyano, methoxy or ethoxy, or represents allyl, C₃-C₆-cycloalkyl, benzyl or phenyl,
- R²: represents C₁-C₄-alkyl which is unsubstituted or substituted by halogen selected from the group consisting of fluorine and/or chlorine, methoxy or ethoxy, or represents C₃-C₄-alkenyl which is optionally substituted by fluorine and chlorine, or represents C₃-C₆-cycloalkyl, or represents benzyl which is unsubstituted or substituted by a compound selected from the group consisting of fluorine, chlorine and methyl, and
- R³: represents the group 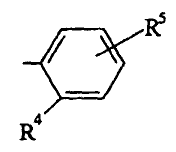
wherein
- R⁴: represents fluorine, chlorine, bromine, methyl, trifluoromethyl, methoxy, difluoromethoxy, trifluoromethoxy, 2-chloro-ethoxy, 2-methoxy-ethoxy, C₁-C₃-alkythio, C₁-C₃-alkysulfinyl, C₁-C₃-alkylsulfonyl, dimethylaminosulfonyl, diethylaminosulfonyl, N-methoxy-N-methylaminosulfonyl, methoxyaminosulfonyl, phenyl, phenoxy or C₁-C₃-alkoxy-carbonyl and
- R⁵: represents hydrogen, fluorine, chlorine or bromine;
furthermore
- R³: represents the radical 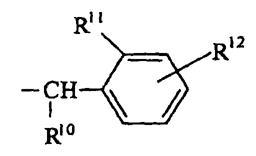
wherein
- R¹⁰: represents hydrogen,
- R¹¹: represents fluorine, chlorine, bromine, methyl, methoxy, difluoromethoxy, trifluorormethoxy, ethoxy, methoxycarbonyl, ethoxycarbonyl, methylsulfonyl or dimethylaminosulfonyl, and
- R¹²: represents hydrogen;
furthermore
- R³: represents the radical 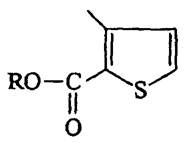
wherein
- R: represents C₁-C₄-alkyl, or represents the radical 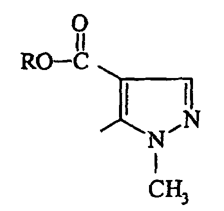
wherein
- R: represents C₁-C₄-alkyl.

The intermediate of general formula (III) used in this process can be obtained in a previous reaction step with or without isolation by reaction of a triazolinone compound of the general formula (I) 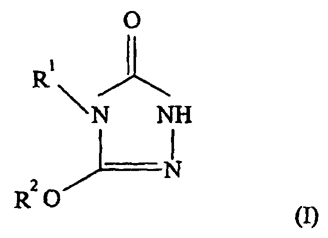 wherein
R¹ and R² each are defined as above,
with a sulfonyl isocyanate of the following general formula (II)

O=C=N-SO₂-R³ (II)

wherein
- R³: is as defined as above.

In a preferred embodiment of the pH-controlled process variant the reaction step for the preparation of the intermediate of the general formula (III) is carried out before the pH-controlled salt production step without isolating the intermediate product of general formula (III).

The novel pH-controlled reaction step is especially well suited for the preparation of the sodium, potassium, magnesium, calcium, ammonium, C₁-C₄-alkyl-ammonium, di-(C₁-C₄-alkyl)-ammonium, tri-(C₁-C₄-alkyl)-ammonium, C₅- or C₆-cycloalkylammonium and di-(C₁-C₂-alkyl)-benzyl-ammonium salts of the compounds according to general formula (IV).

The pH-controlled process step according to the invention is generally carried out at atmospheric pressure. However, it is also possible to conduct the process under elevated or reduced pressure.

The preparation of the compound of general formula (III) by reaction of the substituted triazolinone of the general formula (17 with the sulfonyl isocyanate of the general formula (II) is carried out at a temperature of from about -20°C to about 120°C, and preferably at a temperature of from about 0°C to about 45°C.

The reaction time to produce the intermediate product is up to about 48 hours, and preferably from about 1 hour to about 8 hours.

Suitable sulfonyl isocyanates for producing the intermediate of formula (III) include 2-(trifluoromethoxy) benzensulfonyl isocyanate, 2-(methoxycarbonyl)benzenesulfonyl isocyanate, benzenesulfonyl isocyanate, ρ-toluenesulfonyl isocyanate, 2-fluoro, 2-chloro-, 2-bromo-, 2-methyl-, 2-methoxy-, 2-trifluoromethyl-, 2-difluoromethoxy-, 2-trifluoro-methoxy-, 2-methylthio-, 2-ethylthio-, 2-propylthio-, 2-methylsulfinyl-, 2-methyl-sulfonyl-, 2-dimethylaminosulfonyl-, 2-diethylamino-sulfonyl-, 2-(N-methoxy-N-methyl-aminosulfonyl-, 2-phenyl-, 2-phenoxy-, 2-methoxycarbonyl-, 2-ethoxycarbonyl, 2-propoxycarbonyl- and 2-isopropoxycarbonylphenylsulfonyl isocyanate, 2-fluoro-, 2-chloro-, 2-difluoromethoxy-, 2-trifluoro-methoxy-, 2-methoxycarbonyl- and 2-ethoxycarbonyl-benzylsulfonyl isocyanate, 2-methoxycarbonyl-3-thienyl-sulfonyl isocyanate, 4-methoxycarbonyl- and 4-ethoxycarbonyl-1-methyl-pyrazol-5-yl-sulfonyl isocyanate.

Preferably, the sulfonyl isocyanate is 2-(trifluoromethoxy)benzenesulfonyl isocyanate or 2-(methoxycarbonyl)benzenesulfonyl isocyanate.

The reaction of the substituted triazolinone (formula I) with the sulfonyl isocyanate (formula II) is carried out in the presence of a solvent. Suitable solvents include inert organic solvents such as aliphatic and aromatic, unhalogenated or halogenated hydrocarbons such as pentane, hexane, heptane, cyclohexane, methylene chloride, ethylene chloride, benzene, toluene, xylene, chlorobenzene, dichlorobenzene, petroleum ether, hexane, cyclohexane, dichloromethane, chloroform, carbon tetrachloride; ethers such as diethyl ether, diisopropyl ether, dibutyl ether, dioxane, tetrahydrofuran or diglycol dimethyl ether, glycol dimethyl ether or ethylene glycol diethyl ether; ketones such as acetone, butanone, methyl ethyl ketone, methyl isopropyl ketone or methyl isobutyl ketone; nitriles such as acetonitrile, propionitrile or butyronitrile; amides such as N,N-dimethylformamide, N,N-dimethylacetamide, N-methyl-formanilide, N-methyl-pyrrolidone or hexamethylphosphoric triamide; esters such as methyl acetate or ethyl acetate; also dimethyl sulfoxide, tetramethylene sufone and hexamethylphosphoric triamide.

Preferably, the solvent used in the process of the invention is methyl isobutyl ketone, or xylene, or a commercially available mixture of xylenes containing ortho-xylene, para-xylene and meta-xylene.

The reaction of a sulfonylaminocarbonyl triazolinone product of general formula (III) with a base, to convert the triazolinone product to a salt thereof (final product of the general formula IV) under pH-controlled conditions, is carried out at a temperature of from about -20°C to about 120°C, and preferably from about 0°C to about 45°C.

The reaction time for this conversion to the final product is up to about 48 hours, and preferably from about 2 hours to about 8 hours.

During this conversion of the sulfonylaminocarbonyl triazolinone product of general formula (III) to a salt thereof (final product of the general formula IV), the reaction is carried out under pH controlled conditions. Thus, the base is added to the reaction mixture in an amount such that the pH of the mixture attained is from about 5 to about 10, preferably from about 5.5 to about 9, and most preferably from about 6 to about 7.

Suitable bases for use in this conversion step include bases such as sodium hydroxide, potassium hydroxide, ammonia, or aqueous mixtures thereof. A preferred base is sodium hydroxide, or an aqueous solution of sodium hydroxide.

In an embodiment of the invention, the conversion of the triazolinone product of general formula (III) to the final product of general formula (IV) is carried out in the presence of a solvent. Suitable solvents include aliphatic, alicyclic or aromatic, unhalogenated or halogenated hydrocarbons such as benzene, toluene, xylene, chlorobenzene, dichlorobenzene, petroleum ether, hexane, cyclohexane, dichloromethane, chloroform, tetrachloromethane; ethers such as diethyl ether, diisopropyl ether, dioxane, tetrahydrofuran or ethylene glycol dimethyl ether or ethylene glycol diethyl ether; ketones such as acetone, butanone, or methyl isobutyl ketone; nitriles such as acetonitrile, propionitrile or benzonitrile; amides such as N,N-dimethylformamide, N,N-dimethylacetamide, N-methylformanilide, N-methyl-pyrrolidone or hexamethylphosphoric triamide; esters such as methyl acetate or ethyl acetate, sulfoxides such as dimethyl sulfoxide, alcohols such as methanol, ethanol, n- or i-propanol, n-, i-, s-, or t-butanol, ethylene glycol monomethyl ether, ethylene glycol monoethyl ether, diethylene glycol monomethyl ether, diethylene glycol monoethyl ether; water and mixtures thereof. Preferred solvents include water, methyl isobutyl ketone, propanol, methanol, toluene, a commercially available mixture of xylenes containing ethylbenzene, ortho-xylene, para-xylene, meta-xylene, and mixtures thereof.

In a preferred embodiment of the pH-controlled reaction, the conversion of the intermediate product of general formula (III) to the final product of general formula (IV) is carried out in a mixture of water and methyl isobutyl ketone, or a mixture of water, methanol and xylenes.

The pH-controlled reaction is especially well suited for the conversion 4,5-dihydro-3-methoxy-4-methyl-5-oxo-N-[[2-(trifluoromethoxy)phenyl]sulfonyl]-1H-1,2,4-triazole-1-carboxamide (MSU) to a salt thereof, preferably to the sodium salt with the common name Flucarbazone-sodium.

Further, it is well suited for the conversion of 2-[[[(4,5-dihydro-4-methyl-5-oxo-3-propoxy-1H-1,2,4-triazol-1-yl)carbonyl]amino]sulfonyl)-benzoic acid methyl ester (PSU) to a salt thereof, preferably to the sodium salt with the common name Propoxycarbazone-sodium.

In another embodiment of the pH-controlled reaction step, the salt of the MSU is isolated as a monohydrate.

### EXAMPLES

### Example 2 - The Preparation of Propoxycarbazone-sodium

To a solution (dried by azeotropic distillation) containing 62.8 grams (0.40 moles) of 5-propoxy-4-methyl-2,4-dihydro-3H-1,2,4-triazol-3-one (PMT) in about 250 grams of methyl isobutyl ketone (MIBK) was added a solution of 160.8 grams (0.40 moles) of 60% pure 2-methoxycarbonylbenzenesulfonyl isocyanate in 80 grams of MIBK at a temperature of from about 30°C to about 32°C, over a time period of about 2 hours.

After the addition was complete, the reaction mixture was allowed to come to room temperature and the mixture was stirred for about 16 hours to complete the formation of PSU.

To convert the PSU to Propoxycarbazone-sodium, the reaction mixture was diluted with 10 grams of water and then treated with 50% aqueous NaOH solution over a period of about 6 hours, until all the PSU was converted into its sodium salt, i.e., Propoxycarbazone-sodium.

To isolate the product, the reaction mixture was filtered under vacuum, washed twice with 400 grams of warm (45°C) MIBK and dried under vacuum at room temperature to give 160.8 grams (87% pure) Propoxycarbazone-sodium. The net isolated yield based on PMT was 83.3%.

The purity of the isolated Propoxycarbazone-sodium was only 87% mainly because the isocyanate contained about 15-20% of 2-methoxycarbonyl- benzenesulfonyl chloride as impurity. Some of this hydrolyzed under the reaction conditions to the corresponding sulfonic acid which then formed the sodium salt and ended up in the product.

### Example 3 - The Preparation of Propoxycarbazone-sodium - PSU Isolated

In a 1000 ml flask with mechanical stirrer and thermometer, 119.8 grams (0.2 moles) of isolated PSU and 200 ml of methyl isobutyl ketone (MIBK) were charged. The mixture was agitated and then 10 ml of water was added. The initial pH of the reaction mixture was 2.8. Then 50% sodium hydroxide (NaOH) was added to ambient temperature over a 2 hour time period, at pH controlled conditions. During this addition step, the pH of the reaction mixture was from about 4.6 to about 4.8. The pH of the reaction mixture stabilized at about 7.7. The mixture was agitated from about 1 hour and the solids were isolated by vacuum filtration. The net yield based on PSU was 97.5%.

### Example 4. The Preparation of Flucarbazone-sodium hydrate - MSU Not Isolated

261.0 grams (1.74 moles) of 98% pure 5-methoxy-4-methyl-2,4-dihydro-3H-1,2,4-triazol-3-one (MMT) hydrate in about 2600 grams of methyl isobutyl ketone (MIBK) was dried by azeotropic distillation of part of the MIBK under reduced pressure (with a pot temperature of about 80°C) and then cooled to room temperature under a nitrogen atmosphere. To the resulting MMT slurry in MIBK was added 534.0 grams (1.74 moles) of 87.0% pure 2-(trifluoromethoxy) benzenesulfonyl isocyanate over a period of about 2 hours. The reaction mixture was stirred at room temperature for about 6 hours. The conversion reaction of the MMT (to MSU) was monitored using a liquid chromatograph. About 140.0 grams of water were added to the reaction mixture. The mixture was then treated with 50% aqueous sodium hydroxide (NaOH). The NaOH was added over a period of about 4 hours in an amount such that the pH of the reaction mixture was between about 6 and about 7. The reaction mixture was stirred for about 1 hour. The mixture was then filtered, and washed with about 500 grams of MIBK and dried to isolate the Flucarbazone-sodium hydrate. The yield of Flucarbazone-sodium hydrate was about 93.0% based on MMT hydrate. The purity (water free) was about 98.0%.

### Example 4A

The procedure described in Example 4 was carried out with the exception that the 50% aqueous NaOH was added in an amount such that the pH of the reaction mixture was between about 10.2 and about 10.5. The purity of the Flucarbazone-sodium hydrate decreased to about 96.5%.

### Example 4B

The procedure described in Example 4 was carried out with the exception that the 50% aqueous NaOH was added in an amount such that the pH of the reaction mixture was between about 11.0 and about 11.5. The purity of the Flucarbazone-sodium hydrate decreased to about 95.6%.

### Example 4C

The procedure described in Example 4 was carried out with the exception that the 50% aqueous NaOH was added in an amount such that the pH of the reaction mixture attained was between about 12.0 and about 12.5. The purity of the Flucarbazone-sodium hydrate decreased to about 95.1%.

In Examples 4A, 4B, and 4C, concentrated H₂SO₄ was added to the reaction mixture in an amount such that the pH was about 7.0. The purity of the MKH then increased to about 98.0%.

## Claims

1. A process for preparing a salt of a sulfonylaminocarbonyl triazolinone comprising the step of reacting an intermediate product of general formula (III) 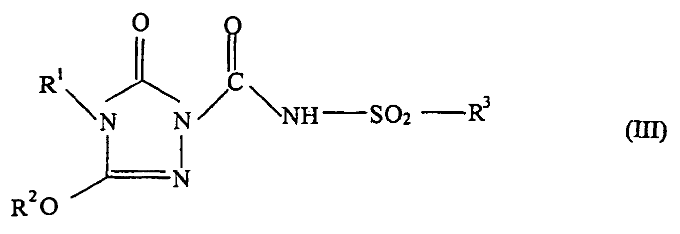 wherein
R¹ represents alkyl, alkenyl or alkynyl having in each case up to 6 carbon atoms, and each of which is unsubstituted or substituted by cyano, halogen, or C₁-C₄-alkoxy,
or
represents cycloalkyl group having 3 to 6 carbon atoms or cycloalkylalkyl group having 3 to 6 carbon atoms in the cycloalkyl moiety and 1 to 4 carbon atoms in the alkyl moiety, each of which is unsubstituted or substituted by halogen or C₁-C₄-alkyl,
or
represents aryl group having 6 or 10 carbon atoms or arylalkyl group having 6 or 10 carbon atoms in the aryl moiety and 1 to 4 carbon atoms in the alkyl moiety, each of which is unsubstituted or substituted by carboxyl, nitro, cyano, halogen, C₁-C₄-alkyl, C₁-C₄-halogenoalkyl, C₁-C₄-alkoxy, C₁-C₄-halogenoalkoxy or C₁-C₄-alkoxycarbonyl,
R² represents alkyl, alkenyl or alkynyl, each of which has up to 6 carbon atoms, and each of which is unsubstituted or substituted by cyano, halogen or C₁-C₄-alkoxy,
or
represents cycloalkyl having 3 to 6 carbon atoms or cycloalkylalkyl having 3 to 6 carbon atoms in the cycloalkyl moiety and 1 to 4 carbon atoms in the alkyl moiety, each of which is unsubstituted or substituted by halogen or C₁-C₄ -alkyle,
or
represents aryl having 6 to 10 carbon atoms or arylalkyl having 6 or 10 carbon atoms in the aryl moiety and 1 to 4 carbon atoms in the alkyl moiety, each of which is unsubstituted or substituted by carboxyl, cyano, nitro, halogen, C₁-C₄-alkyl, C₁-C₄-halogenoalkyl, C₁-C₄-alkoxy, C₁-C₄-halogenoalkoxy or C₁-C₄-alkoxy-carbonyl, and
R³ represents the group 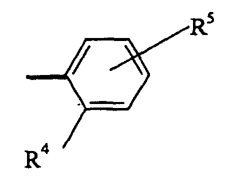 wherein
R⁴ and R⁵ are identical or different and represent hydrogen, fluorine, chlorine, bromine, iodine, nitro, C₁-C₆-alkyl, which is unsubstituted or substtuted by fluorine, chlorine, bromine, cyano, carboxyl, C₁-C₄-alkoxycarbonyl, C₁-C₄-alkylaminocarbonyl, di-(C₁-C₄-alkyl)aminocarbonyl, hydroxyl, C₁-C₄-alkoxy, fonnyloxy, C₁-C₄-alkylcarbonyloxy, C₁-C₄-alkoxycarbonyloxy, C₁-C₄-alkylaminocarbonyloxy, C₁-C₄-alkylthio, C₁-C₄-alkylsulfinyl, C₁-C₄-alkylsulfonyl, di-(C₁-C₄-alkyl)-aminosulfonyl, C₃-C₆-cycloalkyl or phenyl,
or
represent C₂-C₆-alkenyl which is unsubstituted or substituted by fluorine, chlorine, bromine, cyano, C₁-C₄-alkoxycarbonyl, carboxyl or phenyl,
or
represent C₂-C₆-alkynyl which is unsubstituted or substituted by fluorine, chlorine, bromine, cyano, C₁-C₄-alkoxycarbonyl, carboxyl or phenyl,
or
represent C₁-C₄-alkoxy which is unsubstituted or substituted by fluorine, chlorine, bromine, cyano, carboxyl, C₁-C₄-alkoxycarbonyl, C₁-C₄-alkoxy, C₁-C₄-alkylthio, C₁-C₄- alkylsulfinyl or C₁-C₄-alkylsulfonyl,
or
represent C₁-C₄-alkylthio which is unsubstituted or substituted by fluorine, chlorine, bromine, cyano, carboxyl, C₁-C₄-alkoxycarbonyl, C₁-C₄-alkylthio, C₁-C₄ -alkylsulfinyl or C₁-C₄-alkylsulfonyl,
or
represent C₃-C₆ alkenyloxy which is unsubstituted or substituted by fluorine, chlorine, bromine, cyano or C₁-C₄-alkoxycarbonyl,
or
represent C₂-C₆-alkenylthio which is unsubstituted or substituted by fluorine, chlorine, bromine, cyano, nitro, C₁-C₃-aklylthio or C₁-C₄-alkoxycarbonyl, C₃-C₆-alkynyloxy, C₃-C₆-alkynylthio or the radical -S(O)ₚ-R⁶ where p represents the numbers 1 or 2, and
R⁶ represents C₁-C₄-alkyl which is unsubstituted or substituted by fluorine, chlorine, bromine, cyano or C₁-C₄-alkoxy-carbonyl, C₃-C₆-alkenyl, C₃-C₆-alkynyl, C₁-C₄-alkoxy, C₁-C₄-alkoxy-C₁-C₄-alkylamino, C₁-C₄-alkylamino, di-(C₁-C₄-alkyl)amino, phenyl or the radical -NHOR⁷
wherein
R⁷ represents C₁-C₁₂-alkyl which is unsubstituted or substituted by fluorine, chlorine, cyano, C₁-C₄-alkoxy, C₁-C₄-alkylthio, C₁-C₄-alkylsulfinyl, C₁-C₄-alkylsulfonyl, C₁-C₄-alkylcarbonyl, C₁-C₄-alkoxy-carbonyl, C₁-C₄-alkylamino-carbonyl or di-(C₁-C₄-alkyl)-amino-carbonyl), or represents C₃-C₆-alkenyl which is unsubstituted or substituted by fluorine, chlorine or bromine, C₃-C₆-alkynyl, C₃-C₆-cycloalkyl, C₃-C₆-cycloalkyl-C₁-C₂-alkyl, phenyl-C₁-C₂-alkyl which is unsubstituted or substituted by fluorine, chlorine, nitro, cyano, C₁-C₄-alkyl, C₁-C₄-alkoxy or C₁-C₄-alkoxy-carbonyl), or represents benzhydryl, or represents phenyl which is unsubstituted or substituted by fluorine, chlorine, nitro, cyano, C₁-C₄-alkyl, trifluoromethyl, C₁-C₄-alkoxy, C₁-C₂-fluoroalkoxy, C₁-C₄-alkylthio, trifluoromethylthio or C₁-C₄-alkoxy-carbonyl,
R⁴ and/or R⁵ furthermore represent phenyl or phenoxy, or represent C₁-C₄-alkylcarbonylamino, C₁-C₄ -alkoxycarbonyl-amino, C₁-C₄-alkylamino-carbonyl-amino, di-(C₁-C₄-alkyl)-amino-carbonylamino, or the radical -CO-R⁸
wherein
R⁸ represents C₁-C₆-alkyl, C₁-C₆-alkoxy, C₃-C₆-cycloalkoxy, C₃-C₆-alkenyloxy, C₁-C₄-alkylthio, C₁-C₄-alkylamino, C₁-C₄-alkoxyamino, C₁-C₄-alkoxy-C₁-C₄-alkyl-amino or di-(C₁-C₄-alkyl)-amino which are unsubstituted or substituted by halogen selected from the group consisting of fluorine and chlorine,
R⁴ and/or R⁵ furthermore represent trimethylsilyl, thiazolinyl, C₁-C₄-alkylsulfonyloxy, di-(C₁-C₄-alkyl)-aminosulfonylamino or the radical -CH=N-R⁹
wherein
R⁹ represents C₁-C₆-alkyl which is unsubstituted or substituted by fluorine, chlorine, cyano, carboxyl, C₁-C₄-alkoxy, C₁-C₄-alkylthio, C₁-C₄-alkylsulfinyl or C₁-C₄-alkylsulfonyl, or represents benzyl which is unsubstituted or substituted by fluorine or chlorine, or represents C₃-C₆-alkenyl or C₃-C₆-alkynyl, each of which is unsubstituted or substituted by fluorine or chlorine, or represents phenyl which is unsubstituted or substituted by fluorine, chlorine, bromine, C₁-C₄-alkyl, C₁-C₄-alkoxy, trifluoromethyl, trifluoromethoxy or trifluoromethylthio, or represents unsubstituted or halogen substituted C₁-C₆-alkoxy, C₃-C₆-alkenoxy, C₃-C₆-alkynoxy or benzyloxy, wherein the halogen is selected from the group consisting of fluorine and chlorine, or represents amino, C₁-C₄-alkylamino, di-(C₁-C₄-alkyl)-amino, phenylamino, C₁-C₄-alkyl-carbonylamino, C₁-C₄-alkoxy-carbonyl amino or C₁-C₄-alkyl-sulfonyl amino, or represents phenylsulfonylamino which is unsubstituted or substituted by fluorine, chlorine, bromine or methyl,
furthermore
R³ represents the radical 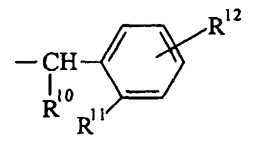 wherein
R¹⁰ represents hydrogen or C₁-C₄-alkyl,
wherein
R¹¹ and R¹² are identical or different and represent hydrogen, fluorine, chlorine, bromine, nitro, cyano, C₁-C₄-alkyl which is unsubstituted or substituted by halogen selected from the group consisting of fluorine and chlorine, C₁-C₄-alkoxy which is unsubstituted or substituted by halogen selected from the group consisting of fluorine and chlorine, carboxyl, C₁-C₄-alkoxycarbonyl, dimethylaminocarbonyl, C₁-C₄-alkylsulfonyl or di-(C₁ -C₄-alkyl)-aminosulfonyl;
furthermore
R³ represents the radical 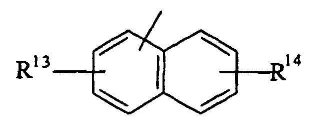 wherein
R¹³ and R¹⁴ are identical or different and represent hydrogen, fluorine, chlorine, bromine, nitro, cyano, C₁-C₄-alkyl which is unsubstituted or substituted by halogen selected from the group consisting of fluorine and chlorine, or C₁-C₄-alkoxy which is unsubstituted or substituted by halogen selected from the group consisting of fluorine and chlorine;
furthermore
R³ represents the radical 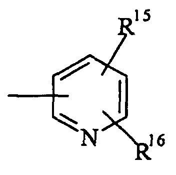 wherein
R¹⁵ and R¹⁶ are identical or different and represent hydrogen, fluorine, chlorine, bromine, nitro, cyano, C₁-C₄-alkyl which is unsubstituted or substituted by halogen selected from the group consisting of fluorine and chlorine, C₁-C₄-alkoxy which is unsubstituted or substituted by halogen selected from the group consisting of fluorine and chlorine, or represent C₁-C₄-alkylthio, C₁-C₄-alkylsulfinyl or C₁-C₄-alkylsulfonyl which are unsubstituted or substituted by halogen selected from the group consisting of fluorine and chlorine, or represent aminosalfonyl, mono-(C₁-C₄-alkyl)-aminosulfonyl, di-(C₁-C₄-alkyl)-aminosulfonyl or C₁-C₄-alkoxycarbonyl or dimethylaminocarbonyl;
furthermore
R³ represents the radical 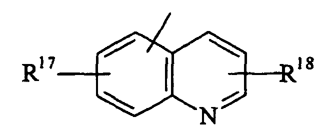 wherein
R¹⁷ and R¹⁸ are identical or different and represent hydrogen, fluorine, chlorine, bromine, C₁-C₄-alkyl which is unsubstituted or substituted by halogen selected from the group consisting of fluorine and bromine, C₁-C₄-alkoxy which is unsubstituted or substituted by halogen selected from the group consisting of fluorine and chlorine, C₁-C₄-alkylthio, C₁-C₄-alkylsulfinyl or C₁-C₄-alkylsulfonyl which are unsubstituted or substituted by halogen selected from the group consisting of fluorine and chlorine, or represent di-(C₁-C₄-alkyl)-aminosulphonyl;
furthermore
R³ represents the radical 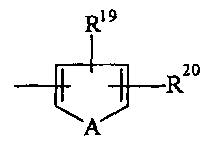 wherein
R¹⁹ and R²⁰ are identical or different and represent hydrogen, fluorine, chlorine, bromine, cyano, nitro, C₁-C₄-alkyl which is unsubstituted or substituted by halogen selected from the group consisting of fluorine and chlorine, C₁-C₄-alkoxy which is unsubstituted or substituted by halogen selected from the group consisting of fluorine and chlorine, C₁-C₄-alkylthio, C₁-C₄-alkylsulfinyl or C₁-C₄-alkysulfonyl which is unsubstituted or substituted by halogen selected from the group consisting of fluorine and chlorine, di-(C₁-C₄-alkylyaminosulfonyl, C₁-C₄-alkoxy-carbonyl or dimethylaminocarbonyl, and
A represents oxygen, sulfur or the group N-Z¹,
wherein
Z¹ represents hydrogen, C₁-C₄-alkyl which is unsubstituted or substituted by fluorine, chlorine, bromine, or cyano, C₃-C₆-cycloalkyl, benzyl, phenyl which is unsubstituted or substituted by fluorine, chlorine, bromine or nitro, C₁-C₄-alkylcarbonyl, C₁-C₄-alkoxy-carbonyl or di-(C₁-C₄-alkyl)-amino-carbonyl;
furthermore
R³ represents the radical 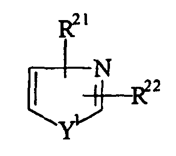 wherein
R²¹ and R²² are identical or different and represent hydrogen, C₁-C₄-alkyl, halogen, C₁-C₄-alkoxycarbonyl, C₁-C₄-alkoxy or C₁-C₄-halogenoalkoxy,
Y¹ represents sulfur or the group N-R²³,
wherein
R²³ represents hydrogen or C₁-C₄-alkyl;
furthermore
R³ represents the radical 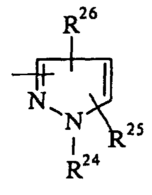 wherein :
R²⁴ represents hydrogen, C₁-C₄-alkyl, benzyl, pyridyl, quinolinyl or phenyl,
R²⁵ represents hydrogen, halogen, cyano, nitro, C₁-C₄-alkyl which is unsubstituted or substituted by halogen selected from the group consisting of fluorine and chlorine, C₁-C₄-alkoxy which is unsubstituted or substituted by halogen selected from the group consisting of fluorine and chlorine, dioxolanyl or C₁-C₄-alkoxy-carbonyl, and
R²⁶ represents hydrogen, halogen or C₁-C₄-alkyl;
furthermore
R³ represents a compound selected from the group consisting of 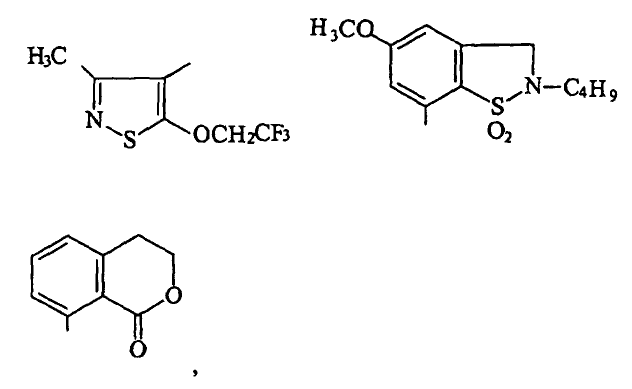 under pH controlled conditions with a base, in such a way that the base is added to the reaction mixture in an amount such that the pH of the mixture attained from 5 to 9, to produce a salt thereof, a final product of the general formula (IV) 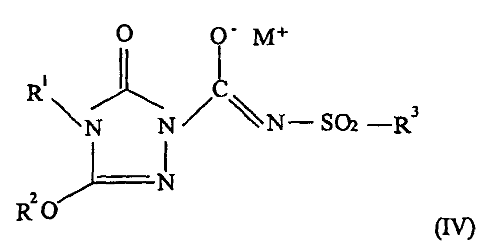 wherein
R¹, R², and R³ are as defined above, and M represents an alkali or alkaline earth metal, or protonated ammonia derivative.

2. The process of Claim 1 which is carried out at a temperature of from about -20°C to about 120°C.

3. The process of Claim 1 which is carried out at a temperature of from about 0°C to about 45°C.

4. The process of Claim 1 wherein the base is selected from the group consisting of sodium hydroxide, potassium hydroxide, ammonia, or aqueous mixtures thereof.

5. The process of Claim 1 wherein 4,5-dihydro-3-methoxy-4-methyl-5-oxo-N-[[2-(trifluoromethoxy)phenyl]sulfonyl]-1 H-1,2,4-triazole-1-carboxamide (MSU) is converted to a salt thereof.

6. The process of Claim 1, wherein 2-[[[(4,5-dihydro-4-methyl-5-oxo-3-propoxy-1H-1,2,4-triazol-1-yl)carbonyl]amino]sulfonyl]benzoic acid methyl ester is converted to a salt thereof.

7. The process of Claim 5 further comprising the step of isolating the MSU salt thereof as a monohydrate.

## Patentansprüche

1. Verfahren zur Herstellung eines Salzes eines Sulfonylaminocarbonyltriazolinons, umfassend den Schritt der Umsetzung eines Zwischenprodukts der allgemeinen Formel (III) 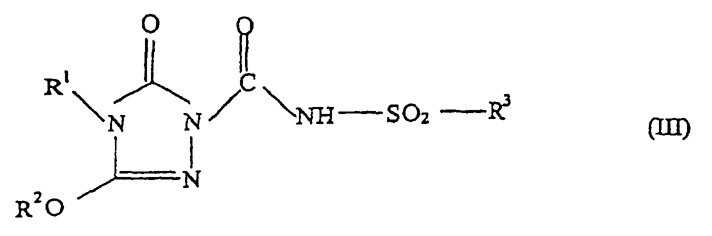 wobei
R¹ ein Alkyl, Alkenyl oder Alkinyl darstellt, das in jedem Fall bis zu 6 Kohlenstoffatome aufweist und jeweils unsubstituiert oder durch Cyan, ein Halogen oder ein C₁-C₄-Alkoxy substituiert ist
oder
eine Cycloalkylgruppe mit 3 bis 6 Kohlenstoffatomen oder eine Cycloalkylalkylgruppe mit 3 bis 6 Kohlenstoffatomen im Cycloal kylrest und 1 bis 4 Kohlenstoffatomen im Alkylrest darstellt, wobei jede davon unsubstituiert oder durch ein Halogen oder ein C₁-C₄-Alkyl substituiert ist,
oder
eine Arylgruppe mit 6 oder 10 Kohlenstoffatomen oder eine Arylalkylgruppe mit 6 oder 10 Kohlenstoffatomen im Arylrest und 1 bis 4 Kohlenstoffatomen im Alkylrest darstellt, wobei jeder davon unsubstituiert oder durch Carboxyl, Nitro, Cyan, ein Halogen, ein C₁-C₄-Alkyl, ein C₁-C₄-Halogenalkyl, ein C₁-C₄-Alkoxy, ein C₁-C₄-Halogenalkoxy oder ein C₁-C₄-Alkoxycarbonyl substituiert ist,
R² ein Alkyl, Alkenyl oder Alkinyl darstellt, das jeweils bis zu 6 Kohlenstoffatome aufweist und jeweils unsubstituiert oder durch Cyan, ein Halogen oder ein C₁-C₄-Alkoxy substituiert ist
oder
ein Cycloalkyl mit 3 bis 6 Kohlenstoffatomen oder ein Cycloalkylalkyl mit 3 bis 6 Kohlenstoffatomen im Cycloalkylrest und 1 bis 4 Kohlenstoffatomen im Alkylrest darstellt, wobei jedes davon unsubstituiert oder durch ein Halogen oder ein C₁-C₄-Alkyl substituiert ist,
oder
ein Aryl mit 6 bis 10 Kohlenstoffatomen oder ein Arylalkyl mit 6 oder 10 Kohlenstoffatomen im Arylrest und 1 bis 4 Kohlenstoffatomen im Alkylrest darstellt, wobei jedes davon unsubstituiert oder durch Carboxyl, Cyan, Nitro, ein Halogen, ein C₁-C₄-Alkyl, ein C₁-C₄-Halogenalkyl, ein C₁-C₄-Alkoxy, ein C₁-C₄-Halogenalkoxy oder ein C₁-C₄-Alkoxycarbonyl substituiert ist, und
R³ die Gruppe 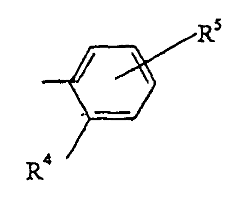 darstellt, wobei
R⁴ und R⁵ gleich oder verschieden sind und Wasserstoff, Fluor, Chlor, Brom, Iod, Nitro, ein C₁-C₆-Alkyl, das unsubstituiert oder durch Fluor, Chlor, Brom, Cyan, Carboxyl, ein C₁-C₄-Alkoxycarbonyl, C₁-C₄-Alkylaminocarbonyl, Di(C₁-C₄-alkyl)aminocarbonyl, Hydroxyl, ein C₁-C₄-Alkoxy, Formyloxy, ein C₁-C₄-Alkylcarbonyloxy, C₁-C₄-Alkoxycarbonyloxy, C₁-C₄-Alkylaminocarbonyloxy, C₁-C₄-Alkylthio, C₁-C₄-Alkylsulfinyl, C₁-C₄-Alkylsulfonyl, Di-(C₁-C₄-alkyl)aminosulfonyl, C₃-C₆-Cycloalkyl oder Phenyl substituiert ist, darstellen,
oder
ein C₂-C₆-Alkenyl darstellen, das unsubstituiert oder durch Fluor, Chlor, Brom Cyan, ein C₁-C₄-Alkoxycarbonyl, Carboxyl oder Phenyl substituiert ist,
oder
ein C₂-C₆-Alkinyl darstellen, das unsubstituiert oder durch Fluor, Chlor, Brom Cyan, ein C₁-C₄-Alkoxycarbonyl, Carboxyl oder Phenyl substituiert ist,
oder
ein C₁-C₄-Alkoxy darstellen, das unsubstituiert oder durch Fluor, Chlor, Brom Cyan, Carboxyl, ein C₁-C₄-Alkoxycarbonyl, C₁-C₄-Alkoxy, C₁-C₄-Alkylthio, C₁-C₄-Alkylsulfinyl oder C₁-C₄-Alkylsulfonyl substituiert ist,
oder
ein C₁-C₄-Alkylthio darstellen, das unsubstituiert oder durch Fluor, Chlor, Brom Cyan, Carboxyl, ein C₁-C₄-Alkoxycarbonyl, C₁-C₄-Alkylthio, C₁-C₄-Alkylsulfinyl oder C₁-C₄-Alkylsulfonyl substituiert ist,
oder
ein C₃-C₆-Alkenyloxy darstellen, das unsubstituiert oder durch Fluor, Chlor, Brom Cyan oder ein C₁-C₄-Alkoxycarbonyl substituiert ist,
oder
ein C₂-C₆-Alkenylthio darstellen, das unsubstituiert oder durch Fluor, Chlor, Brom Cyan, Nitro, ein C₁-C₃-Alkylthio oder C₁-C₄-Alkoxycarbonyl, C₃-C₆-Alkinyloxy, C₃-C₆-Alkinylthio oder den Rest -S(O)ₚ-R⁶, wobei p die Zahlen 1 oder 2 darstellt, substituiert ist, und
R⁶ ein C₁-C₄-Alkyl darstellt, das unsubstituiert oder durch Fluor, Chlor, Brom Cyan, oder ein C₁-C₄-Alkoxycarbonyl, C₃-C₆-Alkenyl, C₃-C₆-Alkinyl, C₃-C₆-Alkoxy, C₁-C₄-Alkoxy-C₁-C₄-alkylamino, C₁-C₄-Alkylamino, Di-(C₁-C₄-alkyl)amino, Phenyl oder den Rest -NHOR⁷ substituiert ist,
wobei
R⁷ ein C₁-C₁₂-Alkyl darstellt, das unsubstituiert oder durch Fluor, Chlor, Cyan, ein C₁-C₄-Alkoxy, C₁-C₄-Alkylthio, C₁-C₄-Alkylsulfinyl, C₁-C₄-Alkylsulfonyl, C₁-C₄-Alkylcarbonyl, C₁-C₄-Alkoxycarbonyl, C₁-C₄-Alkylaminocarbonyl oder Di(C₁-C₄-alkyl)aminocarbonyl) substituiert ist oder ein C₃-C₆-Alkenyl darstellt, das unsubstituiert oder durch Fluor, Chlor oder Brom, ein C₃-C₆-Alkinyl, C₃-C₆-Cycloalkyl, C₃-C₆-Cycloalkyl-C₁-C₂-alkyl, Phenyl-C₁-C₂-alkyl, das unsubstituiert oder durch Fluor, Chlor, Nitro, Cyan, ein C₁-C₄-Alkyl, C₁-C₄-Alkoxy, oder C₁-C₄-Alkoxycarbonyl substituiert ist, substituiert ist oder Benzhydryl darstellt oder ein Phenyl darstellt, das unsubstituiert ist oder durch Fluor, Chlor, Nitro, Cyan, ein C₁-C₄-Alkyl, Trifluormethyl, ein C₁-C₄-Alkoxy, C₁-C₂-Fluoralkoxy, C₁-C₄-Alkylthio, Trifluormethylthio oder ein C₁-C₄-Alkoxycarbonyl substituiert ist,
R⁴ und/oder R⁵ weiterhin Phenyl oder Phenoxy darstellen oder ein C₁-C₄-Alkylcarbonylamino, C₁-C₄-Alkoxycarbonylamino, C₁-C₄-Alkylaminocarbonylamino, Di-(C₁-C₄-alkyl)aminocarbonylamino oder den Rest -COR⁸ darstellen,
wobei
R⁸ ein C₁-C₆-Alkyl, C₁-C₆-Alkoxy, C₃-C₆-Cycloalkyl, C₃-C₆-Alkenyloxy, C₁-C₄-Alkylthio, C₁-C₄-Alkyiamino, C₁-C₄-Alkoxyamino, C₁-C₄-Alkoxy-C₁-C₄-alkylamino oder Di-(C₁-C₄-alkyl)amino, die unsubstituiert oder durch ein aus der aus Fluor und Chlor bestehenden Gruppe ausgewähltes Halogen substituiert ist, darstellt,
R⁴ und/oder R⁵ weiterhin Trimethylsilyl, Thiazolinyl, ein C₁-C₄-Alkylsulfonyfoxy, Di-(C₁-C₄-alkyl)aminosufonylamino oder den Rest -CH=NR⁹ darstellen,
wobei
R⁹ ein C₁-C₆-Alkyl, das unsubstituiert oder durch Fluor, Chlor, Cyan, Carboxyl, ein C₁-C₄-Alkoxy, C₁-C₄-Alkylthio, C₁-C₄-Alkylsulfinyl oder C₁-C₄-Alkylsulfonyl substituiert ist, oder Benzyl darstellt, das unsubstituiert oder durch Fluor oder Chlor substituiert ist oder ein C₃-C₆-Alkenyl oder C₃-C₆-Alkinyl darstellt, das jeweils unsubstituiert oder durch Fluor oder Chlor substituiert ist, oder ein Phenyl darstellt, das unsubstituiert oder durch Fluor, Chlor, Brom, ein C₁-C₄-Alkyl, C₁-C₄-Alkoxy, Trifluormethyl, Trifluormethoxy oder Trifluormethylthio substituiert ist, oder ein unsubstituiertes oder halogensubstituiertes C₁-C₆-Alkoxy, C₃-C₆-Alkenoxy, C₃-C₆-Alkinoxy oder Benzyloxy darstellt, wobei das Halogen aus der aus Fluor und Chlor bestehenden Gruppe ausgewählt ist, oder ein Amino, C₁-C₄-Alkylamino, Di(C₁-C₄-alkyl)amino, Phenylamino, C₁-C₄-Alkylcarbonylamino, C₁-C₄-Alkoxycarbonylamino oder C₁-C₄-Alkylsulfonylamino oder ein Phenylsulfonylamino, das unsubstituiert oder durch Fluor, Chlor, Brom oder Methyl substituiert ist, darstellt,
weiterhin
R³ den Rest 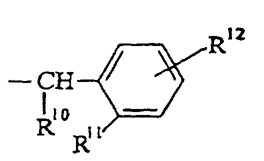 darstellt, wobei
R¹⁰ Wasserstoff oder ein C₁-C₄-Alkyl darstellt,
wobei
R¹¹ und R¹² gleich oder verschieden sind und Wasserstoff, Fluor, Chlor, Brom, Nitro, Cyan, ein C₁-C₄-Alkyl, das unsubstituiert oder durch ein aus der aus Fluor und Chlor bestehenden Gruppe ausgewähltes Halogen substituiert ist, ein C₁-C₄-Alkoxy, das unsubstituiert oder durch ein aus der aus Fluor und Chlor bestehenden Gruppe ausgewähltes Halogen substituiert ist, Carboxyl, ein C₁-C₄-Alkoxycarbonyl, Dimethylaminocarbonyl, C₁-C₄-Alkylsulfonyl oder Di(C₁-C₄-alkyl)aminosulfonyl darstellen,
weiterhin
R³ den Rest 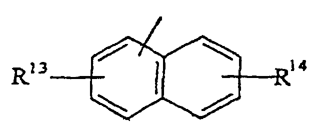 darstellt, wobei
R¹³ und R¹⁴ gleich oder verschieden sind und Wasserstoff, Fluor, Chlor, Brom, Nitro, Cyan, ein C₁-C₄-Alkyl, das unsubstituiert oder durch ein aus der aus Fluor und Chlor bestehenden Gruppe ausgewähltes Halogen substituiert ist, oder ein C₁-C₄-Alkoxy, das unsubstituiert oder durch ein aus der aus Fluor und Chlor bestehenden Gruppe ausgewähltes Halogen substituiert ist, darstellen,
weiterhin
R³ den Rest 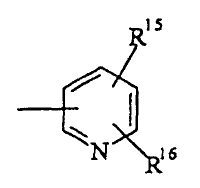 darstellt, wobei
R¹⁵ und R¹⁶ gleich oder verschieden sind und Wasserstoff, Fluor, Chlor, Brom, Nitro, Cyan, ein C₁-C₄-Alkyl, das unsubstituiert oder durch ein aus der aus Fluor und Chlor bestehenden Gruppe ausgewähltes Halogen substituiert ist, ein C₁-C₄-Alkoxy, das unsubstituiert oder durch ein aus der aus Fluor und Chlor bestehenden Gruppe ausgewähltes Halogen substituiert ist, oder ein C₁-C₄-Alkylthio, C₁-C₄-Alkylsulfinyl oder C₁-C₄-Alkylsulfonyl, die substituiert oder durch ein aus der aus Fluor und Chlor bestehenden Gruppe ausgewähltes Halogen substituiert ist, oder Aminosulfonyl, ein Mono(C₁-C₄-alkyl)aminosulfonyl, Di(C₁-C₄-alkyl)aminosulfonyl oder C₁-C₄-Alkoxycarbonyl oder Dimethylaminocarbonyl darstellen,
weiterhin
R³ den Rest 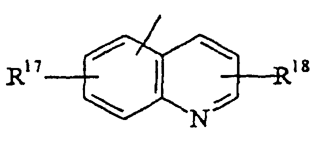 darstellt, wobei
R¹⁷ und R¹⁸ gleich oder verschieden sind und Wasserstoff, Fluor, Chlor, Brom, Nitro, Cyan, ein C₁-C₄-Alkyl, das unsubstituiert oder durch ein aus der aus Fluor und Chlor bestehenden Gruppe ausgewähltes Halogen substituiert ist, ein C₁-C₄-Alkoxy, das unsubstituiert oder durch ein aus der aus Fluor und Chlor bestehenden Gruppe ausgewähltes Halogen substituiert ist, ein C₁-C₄-Alkylthio, C₁-C₄-Alkylsulfinyl oder C₁-C₄-Alkylsulfonyl, die unsubstituiert oder durch ein aus der aus Fluor und Chlor bestehenden Gruppe ausgewähltes Halogen substituiert ist, oder Di(C₁-C₄-alkyl)aminosulfonyl darstellen,
weiterhin
R³ den Rest 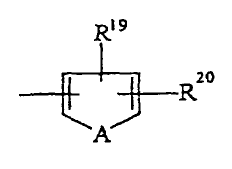 darstellt, wobei
R¹⁹ und R²⁰ gleich oder verschieden sind und Wasserstoff, Fluor, Chlor, Brom, Nitro, Cyan, ein C₁-C₄-Alkyl, das unsubstituiert oder durch ein aus der aus Fluor und Chlor bestehenden Gruppe ausgewähltes Halogen substituiert ist, ein C₁-C₄-Alkoxy, das unsubstituiert oder durch ein aus der aus Fluor und Chlor bestehenden Gruppe ausgewähltes Halogen substituiert ist, ein C₁-C₄-Alkylthio, ein C₁-C₄-Alkylsulfinyl oder C₁-C₄-Alkylsulfonyl, die unsubstituiert oder durch ein aus der aus Fluor und Chlor bestehenden Gruppe ausgewähltes Halogen substituiert ist, oder Di(C₁-C₄-alkyl)aminosulfonyl, C₁-C₄-Alkoxycarbonyl oder Dimethylaminocarbonyl darstellen, und
A Sauerstoff, Schwefel oder die Gruppe N-Z¹ darstellt,
wobei
Z¹ Wasserstoff, ein C₁-C₄-Alkyl, das unsubstituiert oder durch Fluor, Chlor, Brom oder Cyan substituiert ist, ein C₃-C₆-Cyc(oaikyl, Benzyl, Phenyl, das unsubstituiert oder durch Fluor, Chlor, Brom oder Nitro substituiert ist, ein C₁-C₄-Alkylcarbonyl, C₁-C₄-Alkoxycarbonyl oder Di(C₁-C₄-alkyl)aminocarbonyl darstellt,
weiterhin
R³ den Rest 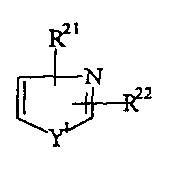 darstellt, wobei
R²¹ und R²² identisch oder verschieden sind und Wasserstoff, ein C₁-C₄-Alkyl, Halogen, C₁-C₄-Alkoxycarbonyl, C₁-C₄-Alkoxy oder C₁-C₄-Halogenalkoxy darstellen,
Y¹ Schwefel oder die Gruppe N-R²³ darstellt,
wobei
R²³ Wasserstoff oder ein C₁-C₄-Alkyl darstellt,
weiterhin
R³ den Rest 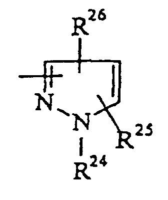 darstellt, wobei
R²⁴ Wasserstoff, ein C₁-C₄-Alkyl, Benzyl, Pyridyl, Chinolinyl oder Phenyl darstellt,
R²⁵ Wasserstoff, ein Halogen, Cyan, Nitro, ein C₁-C₄-Alkyl, das unsubstituiert oder durch ein aus der aus Fluor und Chlor bestehenden Gruppe ausgewähltes Halogen substituiert ist, ein C₁-C₄-Alkoxy, das unsubstituiert oder durch ein aus der aus Fluor und Chlor bestehenden Gruppe ausgewähltes Halogen substituiert ist, Dioxolanyl oder ein C₁-C₄-Alkoxycarbonyl darstellt, und
R²⁶ Wasserstoff, ein Halogen oder ein C₁-C₄-Alkyl darstellt,
weiterhin
R³ eine Verbindung darstellt, die aus der aus 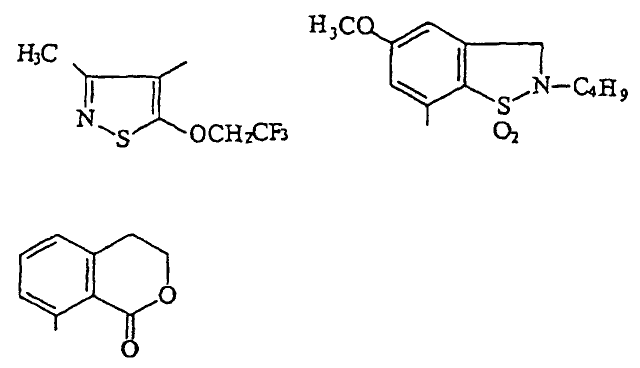
bestehenden Gruppe unter Bedingungen einer Regelung des pH-Werts mit einer Base so ausgewählt ist, dass die Base in einer Menge so zur Reaktionsmischung gegeben wird, dass der pH-Wert der erhaltenen Mischung 5 bis 9 beträgt, wodurch ein Salz davon, ein Endprodukt der allgemeinen Formel (IV) 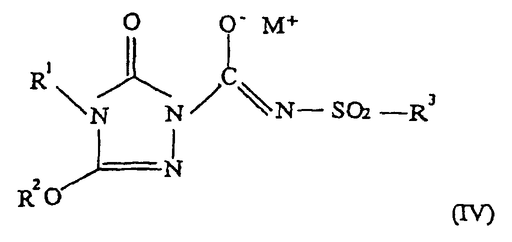 erzeugt wird, wobei
R¹, R² und R³ wie oben definiert sind und M ein Alkali- oder Erdalkalimetall oder ein protoniertes Ammoniakderivat darstellt.

2. Verfahren nach Anspruch 1, das bei einer Temperatur von etwa -20°C bis etwa 120 °C durchgeführt wird.

3. Verfahren nach Anspruch 1, das bei einer Temperatur von etwa 0 °C bis etwa 45 °C durchgeführt wird.

4. Verfahren nach Anspruch 1, wobei die Base aus der aus Natriumhydroxid, Kaliumhydroxid, Ammoniak oder wässrigen Mischungen davon bestehenden Gruppe ausgewählt ist.

5. Verfahren nach Anspruch 1, wobei 4,5-Dihydro-3-methoxy-4-methyl-5-oxo-N-[[2-(Trifluormethoxy)phenyl]sulfonyl]-1H-1,2,4-triazol-1-carboxamid (MSU) zu einem Salz davon umgewandelt wird.

6. Verfahren nach Anspruch 1, wobei 2-[[[(4,5-Dihydro-4-methyl-4-oxo-3-propoxy-1H-1,2,4-triazof-1-yl)carbonyl]amino]sulfonyl]benzoesäuremethylester zu einem Salz davon umgewandelt wird.

7. Verfahren nach Anspruch 5, weiterhin umfassend den Schritt der Isolierung des MSU-Salzes als Monohydrat.

## Revendications

1. Procédé de préparation d'un sel de sulfonylaminocarbonyltriazolinone comprenant l'étape de réaction d'un produit intermédiaire de formule générale (III) : 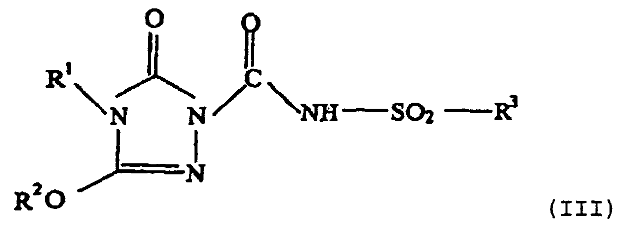 dans laquelle
R¹ représente un alkyle, un alcényle ou un alcynyle, ayant dans chaque cas jusqu'à 6 atomes de carbone, et dont chacun est non substitué ou substitué par un cyano, un halogène ou un alkoxy en C₁-C₄,
ou
représente un groupement cycloalkyle ayant 3 à 6 atomes de carbone ou un groupement cycloalkylalkyle ayant 3 à 6 atomes de carbone dans la partie cycloalkyle, et 1 à 4 atomes de carbone dans la partie alkyle, dont chacun est non substitué ou substitué par un halogène ou un alkyle en C₁-C₄,
ou
représente un groupement aryle ayant 6 ou 10 atomes de carbone ou un groupement arylalkyle ayant 6 ou 10 atomes de carbone dans la partie aryle et 1 à 4 atomes de carbone dans la partie alkyle, dont chacun est non substitué ou substitué par un carboxyle, nitro, cyano, halogène, alkyle en C₁-C₄, halogénoalkyle en C₁-C₄, alkoxy en C₁-C₄, halogénoalkoxy en C₁-C₄ ou alkoxycarbonyle en C₁-C₄,
R² représente un alkyle, un alcényle ou un alcynyle, dont chacun a jusqu'à 6 atomes de carbone, et dont chacun est non substitué ou substitué par un cyano, un halogène ou un alkoxy en C₁-C₄,
ou
représente un cycloalkyle ayant 3 à 6 atomes de carbone ou un cycloalkylalkyle ayant 3 à 6 atomes de carbone dans la partie cycloalkyle, et 1 à 4 atomes de carbone dans la partie alkyle, dont chacun est non substitué ou substitué par un halogène ou un alkyle en C₁-C₄,
ou
représente un aryle ayant 6 à 10 atomes de carbone ou un arylalkyle ayant 6 ou 10 atomes de carbone dans la partie aryle et 1 à 4 atomes de carbone dans la partie alkyle, dont chacun est non substitué ou substitué par un carboxyle, un cyano, un nitro, un halogène, un alkyle en C₁-C₄, un halogénoalkyle en C₁-C₄, un alkoxy en C₁-C₄, un halogénoalkoxy en C₁-C₄ ou un alkoxycarbonyle en C₁-C₄, et
R³ représente le groupement 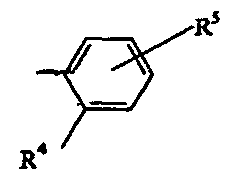 dans lequel
R⁴ et R⁵ sont identiques ou différents et représentent un hydrogène, fluor, chlore, brome, iode, nitro, alkyle en C₁-C₆ qui est non substitué ou substitué par un fluor, chlore, brome, cyano, carboxyle, alkoxycarbonyle en C₁-C₄, alkylaminocarbonyle en C₁-C₄, di(alkyle en C₁-C₄)-aminocarbonyle, hydroxyle, alkoxy en C₁-C₄, formyloxy, alkylcarbonyloxy en C₁-C₄, alkoxycarbonyloxy en C₁-C₄, alkylaminocarbonyloxy en C₁-C, alkylthio en C₁-C₄, alkylsulfinyle en C₁-C₄, alkylsulfonyle en C₁-C₄, di (alkyle en C₁-C₄)amino-sulfonyle, cycloalkyle en C₃-C₆ ou phényle,
ou
représentent un alcényle en C₂-C₆, qui est non substitué ou substitué par un fluor, chlore, brome, cyano, alkoxycarbonyle en C₁-C₄, carboxyle ou phényle,
ou
représentent un alcynyle en C₂-C₆, qui est non substitué ou substitué par un fluor, chlore, brome, cyano, alkoxycarbonyle en C₁-C₄, carboxyle ou phényle,
ou
représentent un alkoxy en C₁-C₄ qui est non substitué ou substitué par un fluor, chlore, brome, cyano, carboxyle, alkoxycarbonyle en C₁-C₄, alkoxy en C₁-C₄, alkylthio en C₁-C₄, alkylsulfinyle en C₁-C₄ ou alkylsulfonyle en C₁-C₄,
ou
représentent un alkylthio en C₁-C₄ qui est non substitué ou substitué par un fluor, chlore, brome, cyano, carboxyle, alkoxycarbonyle en C₁-C₄, alkylthio en C₁-C₄, alkylsulfinyle en C₁-C₄ ou alkylsulfonyle en C₁-C₄,
ou
représentent un alcényloxy en C₃-C₆ qui est non substitué ou substitué par un fluor, chlore, brome, cyano ou alkoxycarbonyle en C₁-C₄,
ou
représentent un alcénylthio en C₂-C₆ qui est non substitué ou substitué par un fluor, chlore, brome, cyano, nitro, alkylthio en C₁-C₃ ou alkoxycarbonyle en C₁-C₄, alcynyloxy en C₃-C₆, alcynylthio en C₃-C₆ ou le radical -S(O)ₚ-R⁶ où p représente les nombres 1 ou 2, et R⁶ représente un alkyle en C₁-C₄ qui est non substitué ou substitué par un fluor, chlore, brome, cyano ou alkoxycarbonyle en C₁-C₄, alcényle en C₃-C₆, alcynyle en C₃-C₆, alkoxy en C₁-C₄, (alkoxy en C₁-C₄)-(alkyle en C₁-C₄) amino, alkyle amino en C₁-C₄, di (alkyle en C₁-C₄) amino, phényle ou radical -NHOR⁷
où
R⁷ représente un alkyle en C₁-C₁₂ qui est non substitué ou substitué par un fluor, chlore, cyano, alkoxy en C₁-C₄, alkylthio en C₁-C₄, alkylsulfinyle en C₁-C₄, alkylsulfonyle en C₁-C₄, alkylcarbonyle en C₁-C₄, alkoxycarbonyl en C₁-C₄, alkylaminocarbonyle en C₁-C₄, ou di (alkyle en C₁-C₄)-aminocarbonyle, ou représente un alcényle en C₃-C₆ qui est non substitué ou substitué par un fluor, chlore ou brome, alcynyle en C₃-C₆, cycloalkyle en C₃-C₆, (cycloalkyle en C₃-C₆) - (alkyle en C₁-C₂) , phénylalkyle en C₁-C₂ qui est non substitué ou substitué par un fluor, chlore, nitro, cyano, alkyle en C₁-C₄, alkoxy en C₁-C₄ ou alkoxycarbonyle en C₁-C₄, ou représente un benzhydryle, ou représente un phényle qui est non substitué ou substitué par un fluor, chlore, nitro, cyano, alkyle en C₁-C₄, trifluorométhyle, alkoxy en C₁-C₄, fluoroalkoxy en C₁-C₂, alkylthio en C₁-C₄, trifluorométhylthio ou alkoxycarbonyle en C₁-C₄,
R⁴ et/ou R⁵ représentent en plus un phényle ou phénoxy ,
ou
représentent un alkylcarbonylamino en C₁-C₄, alkoxycarbonylamino en C₁-C₄, alkylaminocarbonylamino en C₁-C₄, di (alkyle en C₁-C₄) aminocarbonylamino ou le radical -COR⁸
dans lequel
R⁸ représente alkyle en C₁-C₆, alkoxy en C₁-C₆, cycloalkoxy en C₃-C₆, alcényloxy en C₃-C₆, alkylthio en C₁-C₄, alkylamino en C₁-C₄, alkoxyamino en C₁-C₄, (alkoxy en C₁-C₄) (alkylamino en C₁-C₄) ou di (alkyle en C₁-C₄)amino, qui sont non substitués ou substitués par un halogène choisi parmi le groupe constitué dy fluor et du chlore, R⁴ et/ou R⁵ représentent en outre un triméthylsilyle, thiazolinyle, alkylsulfonyloxy en C₁-C₄, di (alkyle en C₁-C₄)aminoasulfonylamino ou le radical -CH=N-R⁹
dans lequel
R⁹ représente un alkyle en C₁-C₆ qui est non substitué ou substitué par un fluor, chlore, cyano, carboxyle, alkoxy en C₁-C₄, alkylthio en C₁-C₄, alkylsulfinyle en C₁-C₄ ou alkylsulfonyle en C₁-C₄, ou représente un benzyle qui est non substitué ou substitué par un fluor ou un chlore, ou représente un alcényle en C₃-C₆ ou un alcynyle en C₃-C₆, dont chacun est non substitué ou substitué par un fluor ou un chlore ou représente un phényle qui est non substitué ou substitué par un fluor, chlore, brome, alkyle en C₁-C₄, alkoxy en C₁-C₄, trifluorométhyle, trifluorométhoxy ou trifluorométhylthio, ou représente un alkoxy en C₁-C₆, alcénoxy en C₃-C₆, alcynoxy en C₃-C₆ ou benzyloxy, non substitué ou substitué par un halogène, où l'halogène est choisi parmi le groupe constitué du fluor et du chlore, ou représente un amino, alkylamino en C₁-C₄, di(alkyle en C₁-C₄)amino, phénylamino, alkylcarbonylamino en C₁-C₄, alkoxycarbonylamiono en C₁-C₄, ou alkylsulfonylamino en C₁-C₄, ou représente un phénylsulfonylamino qui est non substitué ou substitué par un fluor, chlore, brome ou méthyle,
en outre
R³ représente le radical 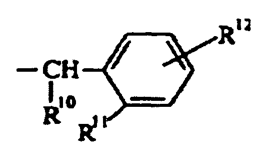 dans lequel
R¹⁰ représente un hydrogène ou un alkyle en C₁-C₄
dans lequel
R¹¹ et R¹² sont identiques ou différentes et représentent un hydrogène, fluor, chlore, brome, nitro, cyano, alkyle en C₁-C₄ qui est non substitué ou substitué par un halogène choisi parmi le groupe constitué du fluor et du chlore, alkoxy en C₁-C₄ qui est non substitué ou substitué par un halogène choisi parmi le groupe constitué du fluor et du chlore, carboxyle, alkoxycarbonyle en C₁-C₄, diméthylaminocarbonyle, alkylsulfonyle en C₁-C₄ ou di(alkyle en C₁-C₄)-aminosulfonyle ;
en outre
R³ représente le radical 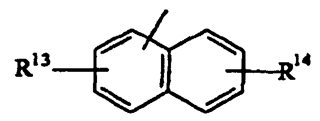 dans lequel
R¹³ et R¹⁴ sont identiques ou différents et représentent un hydrogène, fluor, chlore, brome, nitro, cyano, alkyle en C₁-C₄ qui est non substitué ou substitué par un halogène choisi parmi le groupe constitué du fluor et du chlore, ou alkoxy en C₁-C₄ qui est non substitué ou substitué par un halogène choisi parmi le groupe constitué du fluor et du chlore ;
en outre
R³ représente le radical 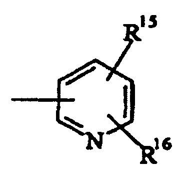 dans lequel
R¹⁵ et R¹⁶ sont identiques ou différents et représentent un hydrogène, fluor, chlore, brome, nitro, cyano, alkyle en C₁-C₄ qui est non substitué ou substitué par un halogène choisi parmi le groupe constitué du fluor et du chlore, alkoxy en C₁-C₄ qui est non substitué ou substitué par un halogène choisi parmi le groupe constitué du fluor et du chlore, ou représentent un alkylthio en C₁-C₄, alkylsulfinyle en C₁-C₄ ou un alkylsulfonyle en C₁-C₄ qui sont non substitués ou substitués par un halogène choisi parmi le groupe constitué du fluor et du chlore, ou représentent un aminosulfonyle, mono- (alkyle en C₁-C₄) aminosulfonyle, di(alkyle en C₁-C₄) aminosulfonyle ou alkoxycarbonyle en C₁-C₄ ou diméthylaminocarbonyle ;
en outre
R³ représente le radical 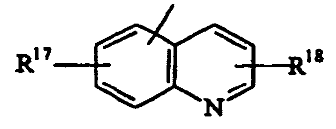 dans lequel
R¹⁷ et R¹⁸ sont identiques ou différents et représentent un hydrogène, fluor, chlore, brome, alkyle en C₁-C₄ qui est non substitué ou substitué par un halogène choisi parmi le groupe constitué du fluor et du brome, alkoxy en C₁-C₄ qui est non substitué ou substitué par un halogène choisi parmi le groupe constitué du fluor et du chlore, alkylthio en C₁-C₄, alkylsulfinyle en C₁-C₄ ou un alkylsulfonyle en C₁-C₄ qui est non substitué ou substitué par un halogène choisi parmi le groupe constitué du fluor et du chlore, ou représentent un di (alkyle en C₁-C₄) aminosulfonyle ;
en outre
R³ représente le radical 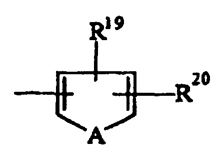 dans lequel
R¹⁹ et R²⁰ sont identiques ou différents et représentent un hydrogène, fluor, chlore, brome, cyano, nitro, alkyle en C₁-C₄ qui est non substitué ou substitué par un halogène choisi parmi le groupe constitué du fluor et du chlore, alkoxy en C₁-C₄ qui est non substitué ou substitué par un halogène choisi parmi le groupe constitué du fluor et du chlore, alkylthio en C₁-C₄, alkylsulfinyle en C₁-C₄ ou un alkylsulfonyle en C₁-C₄ qui est non substitué ou substitué par un halogène choisi parmi le groupe constitué du fluor et du chlore, di(alkyle en C₁-C₄)aminosulfonyle ou alkoxycarbonyle en C₁-C₄ ou diméthylaminocarbonyle, et
A représente un oxygène, un soufre ou le groupement N-Z¹;
dans lequel
Z¹ représente un hydrogène, alkyle en C₁-C₄ qui est non substitué ou substitué par un fluor, chlore, brome, ou cyano, cycloalkyle en C₃-C₆, benzyle, phényle qui est non substitué ou substitué par un fluor, chlore, brome ou nitro, alkylcarbonyle en C₁-C₄, alkoxycarbonyle en C₁-C₄ ou di (alkyle en C₁-C₄) aminocarbonyle ;
en outre
R³ représente le radical 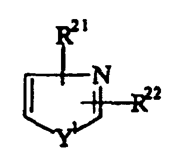 dans lequel
R²¹ et R²² sont identiques ou différents et représentent un hydrogène, alkyle en C₁-C₄, halogène, alkoxycarbonyle en C₁-C₄, alkoxy en C₁-C₄ ou halogénoalkoxy en C₁-C₄,
Y¹ représente un soufre ou le groupement -N-R²³,
dans lequel
R²³ représente un hydrogène ou un alkyle en C₁-C₄ ;
en outre
R³ représente le radical 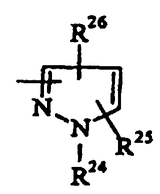 dans lequel
R²⁴ représente un hydrogène, alkyle en C₁-C₄, benzyle, pyridyle, quinolinyle ou phényle,
R²⁵ représente un hydrogène, halogène, cyano, nitro, alkyle en C₁-C₄ qui est non substitué ou substitué par un halogène choisi parmi le groupe constitué du fluor et du chlore, alkoxy en C₁-C₄ qui est non substitué ou substitué par un halogène choisi parmi le groupe constitué du fluor et du chlore, dioxolanyle ou alkoxycarbonyle en C₁-C₄, et
R²⁶ représente un hydrogène, un halogène ou un alkyle en C₁-C₄ ;
en outre
R³ représente un composé choisi parmi le groupe constitué de 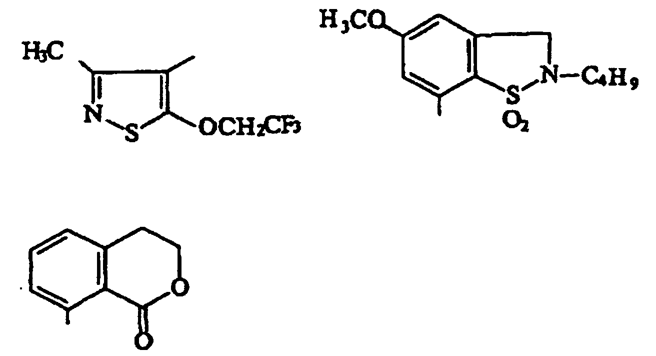 sous des conditions de pH contrôlé avec une base, de telle sorte que la base est ajoutée au mélange de réaction dans une quantité telle que le pH du mélange atteint est de 5 à 9 pour produire un sel de celle-ci, un produit final de formule générale (IV) 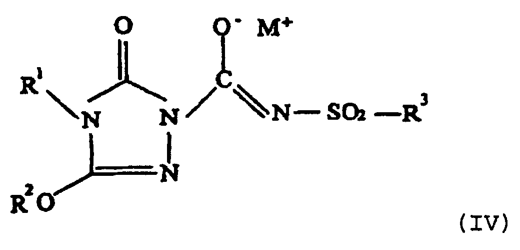 dans laquelle
R¹, R² et R³ sont comme définis ci-dessus et M représente un métal alcalin ou alcalino-terreux ou un dérivé d'ammoniac protoné.

2. Procédé selon la revendication 1 qui est effectué à une température d'environ -20°C à environ 120°C.

3. Procédé selon la revendication 1 qui est effectué à une température d'environ 0°C à environ 45°C.

4. Procédé selon la revendication 1 dans lequel la base est choisie parmi le groupe constitué de l'hydroxyde de sodium, de l'hydroxyde de potassium, de l'ammoniac ou des mélanges de ceux-ci.

5. Procédé selon la revendication 1 dans lequel le 4,5-dihydro-3-méthoxy-4-méthyl-5-oxo-N-[[2-(trifluorométhoxy)phényl]sulfonyl]-1H-1,2,4-triazole-1-carboxamide (MSU) est converti en un sel de celui-ci.

6. Procédé selon la revendication dans lequel l'ester méthylique de l'acide 2-[[[(4,5-dihydro-4-méthyl-5-oxo-3-propoxy-1H-1,2,4-triazol-1-yl}carbonyl]-amino]sulfonyl]benzoïque est converti en un sel de celui-ci.

7. Procédé selon la revendication 5 comprenant en plus l'étape d'isolement du sel de MSU de celui-ci sous forme de monohydrate.
